# EUROPEAN PATENT SPECIFICATION

(11) **EP 2 544 554 B1**
(45) Date of publication and mention of the grant of the patent: **30.11.2016**
(21) Application number: 11708623.1
(22) Date of filing: 01.03.2011
(51) Int. Cl.: A23L 29/212, A23L 29/231, A23L 29/238, A23L 29/25, A23L 29/269, A23L 2/52, A23L 2/66, A23L 33/10

(54) **METHOD FOR STABILIZING WATER INSOLUBLE BIOACTIVE COMPOUND AQUEOUS DISPERSIONS**
VERFAHREN ZUR STABILISIERUNG VON WÄSSRIGEN DISPERSIONEN MIT EINEM WASSERUNLÖSLICHEN BIOWIRKSTOFF
PROCÉDÉ POUR STABILISER DES DISPERSIONS AQUEUSES CONTENANT UN COMPOSÉ BIOACTIF INSOLUBLE DANS L'EAU

(30) Priority: 11.03.2010 US 312694 P
(43) Date of publication of application: 16.01.2013
(73) Proprietor: Stokely-Van Camp, Inc., Chicago, IL 60661 (US)
(72) Inventor: ZHANG, Naijie, Ridgefield CT 06877 (US); RINALDI, Vincent, E., A., Bethel CT 06801 (US); GIVEN JR., Peter, S., Ridgefield CT 06877 (US)
(74) Representative: Duxbury, Stephen
(86) International application number: PCT/US2011/026584
(87) International publication number: WO 2011/112386

(56) References cited:
- EP-A1- 0 543 051
- WO-A1-00/33673
- WO-A2-2008/140065
- WO-A2-2010/013224
- LATIFA CHEBIL, CATHERINE HUMEAU, JULIE ANTHONI, FRANÇOIS DEHEZ, JEAN-MARC ENGASSER, AND MOHAMED GHOUL: "Solubility of Flavonoids in Organic Solvents", J. CHEM. ENG. DATA, vol. 52, no. 5, 2007, pages 1552-1556, XP002639879,
- DATABASE WPI Week 200962 Thomson Scientific, London, GB; AN 2009-N41584 XP002639880, & JP 2009 201371 A (YOKOHAMA YUSHI KOGYO KK) 10 September 2009 (2009-09-10)

## Description

### FIELD OF THE INVENTION

The present invention relates generally to a method for dispersing and stabilizing water insoluble bioactive compounds in a liquid medium and a composition containing flavonoid and a suspension agent.

### BACKGROUND

Biologically active (bioactive) compounds such as flavonoids in general, are used as nutritional supplements to provide, for example, antioxidants. Most of bioactive compounds are highly rigid and crystalline and water insoluble. Quercetin, in particular, is considered a powerful antioxidant. A number of studies showed that quercetin is effective for the prevention of various diseases.

Flavonoids may be extracted from plants. For example, quercetin is a natural, plant-derived, flavonoid. In particular, quercetin is the aglycone form of a number of other flavonoid glycosides, such as rutin and quercitrin, found in citrus fruit, cranberries, blueberries, buckwheat, onions, and other vegetables, fruits, and green plants. The chemical structure of quercetin is illustrated below:

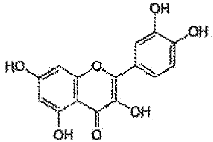

### 2-(3,4-dihydroxyphenyl)-3,5,7-trihydroxy-4H-chromen-4-one

It is desired to use flavonoids as a nutritional supplement in food products such as beverages. Often such flavonoids are difficult to disperse and mix into beverage products at efficacious concentrations. Often such flavonoids will simply settle to the bottom of the container holding the beverage. For example, quercetin is typically obtained as a powder and is insoluble in water. When added to liquid media, quercetin usually agglomerizes and settles to the bottom of the beverage, thereby resulting in a product that is not visually appealing to the consumer.

Chebil et al (2007, "Solubility of flavonoids in organic solvents")relates to the solubility of flavonoids in organic solvents. Discloses is the solubility of quercetin, isoquercetin, rutin, chrysin, naringenin and hesperetin in acetonitrile, acetone and tert-amyl alcohol, in particular at 50°C.

EP0543051 A1 relates to a beverage containing a flavonoid containing plant extract. Specifically disclosed is the preparation of a beverage comprising a flavonoid containing extract which is water soluble, water insoluble of sparingly water soluble. Suitable plant extracts are ginkgo leaf extract, persimmon leaf extract, crataegus leaf extract, Japanese pagoda leaf extract and lyceum fruit extract. These extracts are prepared by extracting such a plant or part thereof with water or aqueous ethanol. The beverage further comprises xanthan gum and water.

WO 2010/022364 discloses a method of forming water soluble microparticles. Oil soluble supplements are dissolved in ethanol or another polar solvent often at elevated temperature, typically at 50°C, to increase their solubility. Antioxidant which may be used as stabilizers or helpers for water insoluble drugs such as proteins or peptide drug are dissolved in a water miscible polar solvent to form a premix which can subsequently be mixed with plain water.

Therefore, a need exists in the food and beverage industry to provide the consumer with a food product containing bioactive compounds such as quercetin wherein the bioactive compound is stabilized in an aqueous suspension.

### BRIEF SUMMARY

The present invention relates to a method for stabilizing suspended quercetin or other suspended water insoluble bioactive compounds in a beverage and a composition for a beverage containing stabilized suspended quercetin or other water insoluble bioactive compounds.

### BRIEF DESCRIPTION OF THE DRAWINGS

Figs. 1A and 1B compare amorphous quercetin and crystalline quercetin, respectively.
Figs. 2A and 2B show the results of a quercetin stability study by FTIR and Carbon-13 NMR, respectively.
Fig. 3 shows samples of stable aqueous dispersions prepared in accordance with the present invention.

### DETAILED DESCRIPTION OF THE INVENTION

The present invention relates to a method for stabilizing suspended microparticulated water insoluble bioactive compounds such as quercetin particles in a liquid medium, such as a beverage, and further to a composition containing solubilized or dispersed compound particles and at least one dispersion stabilizer. Beverages, prepared with solubilized or dispersed a bioactive compound and a dispersion stabilizer, contain fine, stable dispersions.

The water insoluble bioactive compounds may be polyphenols, flavanoids, alkaloids, aldehyde, aryl ketone, benzofuranoid, benzopyranoid, diterpenoid, phenylpropanoid, polyketide, sesquiterpenoid, monoterpenoid, and/or may be derived from plants, herbs, or botanicals. See for example, Naturally Occurring Bioactive Compounds Edited by Mahendra Rai, Maria Cecillia Carpinella, 2006. Bioactive Compounds in Foods Edited by John Gilbert and Hamide Z. Senyuva. Bioactive Compounds From Plants, Volume 154, John Wiley and Sons, 1990.

Suitable polyphenols include quercetin, eriocitrin, neoeriocitrin, narirutin, naringin, hesperidin, hesperetin, neohesperidin, neoponcirin, poncirin, rutin, isorhoifolin, rhoifolin, diosmin, neodiosmin, sinensetin, nobiletin, tangeritin, catechin, catechin gallate, epigallocatechin, epigallocatechin gallate, anthocyanin, heptamethoxyflavone, curcumin, resveratrol, naringenin, tetramethoxyflavone, kaempferol, and rhoifolin,

Suitable flavonoids and other bioactive compounds include quercetin, flavonones, flavones, dihydroflavonols, flavonols, flavandiols, leucoanthocyanidins, flavonol glycosodes, flavonone glycosides, isoflavonoids, and neoflavonoids. In particular, the favonoids may be, but not limited to, quercetin, eriocitrin, neoeriocitrin, narirutin, naringin, hesperidin, hesperetin, neohesperidin, neoponcirin, poncirin, rutin, isorhoifolin, rhoifolin, diosmin, neodiosmin, sinensetin, nobiletin, tangcritin, catechin, catechin gallate, epigallocatechin, epigallocatechin gallate, oolong tea polymerized polyphenol, anthocyanin, heptamethoxyflavone, daidzin, daidzein, biochaminn A, prunetin, genistin, glycitein, glycitin, genistein, 6,7,4' trihydroxy isoflavone, morin, apigenin, vitexin, balcalein, apiin, cupressuflavone, datiscetin, diosmetin, fisetin, galangin, gossypetin, geraldol, hinokiflavone, primuletin, pratol, luteolin, myricetin, orientin, robinetin, quercetagetin, and hydroxy-4-flavone.

Suitable compounds derived from plants, herbs, or botanicals include pyrrolizldine, alkaloids, and artemisinin.

For ease of discussion, the application will be discussed in terms of quercetin. However, it is intended that the process steps and compositions apply to all suitable bioactive compounds described above and below.

In one aspect of the invention, quercetin is solubilized or dispersed prior to adding the quercetin to an aqueous solution containing a dispersion stabilizer.

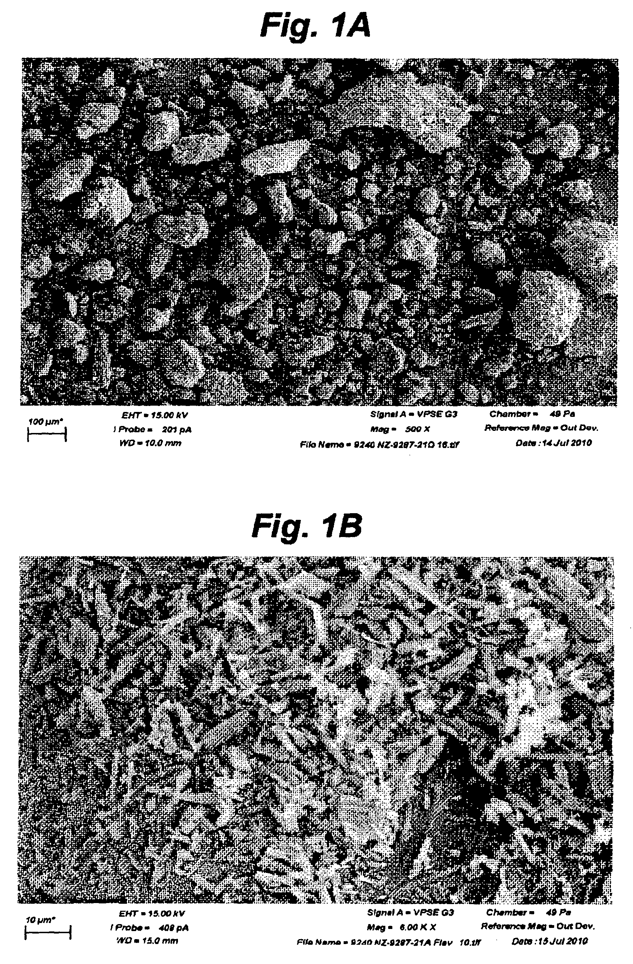

An aqueous dispersion of quercetin through solubilization reduces particle size, density, rigidity, and crystalline structure resulting in increasing dispersion stability. SEM results show dispersed quercetin in gellan gum has amorphous structure (Fig. 1A) compared to crystalline quercetin (Fig. 1B),

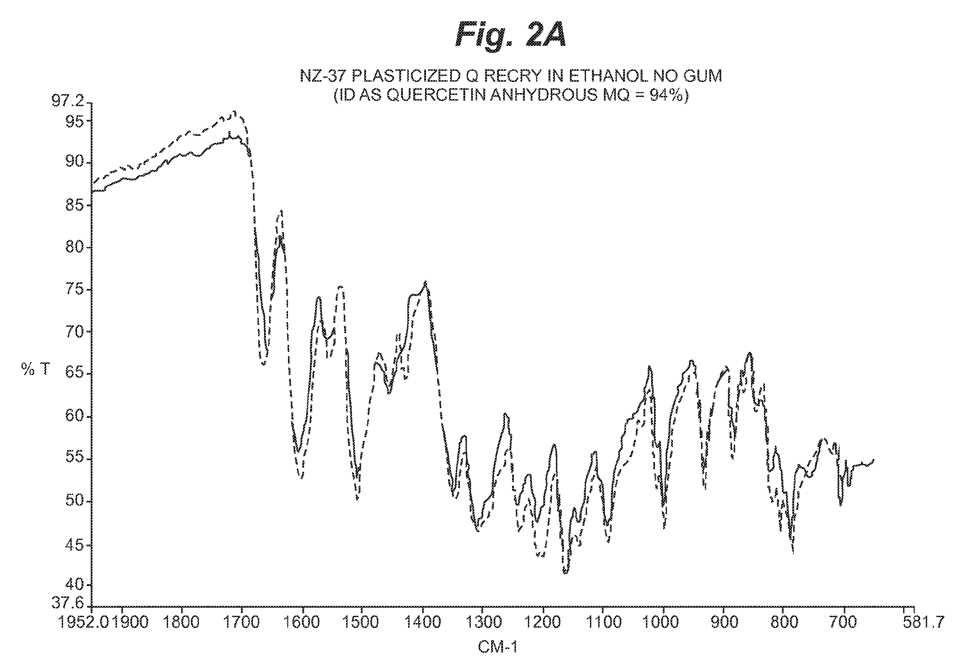

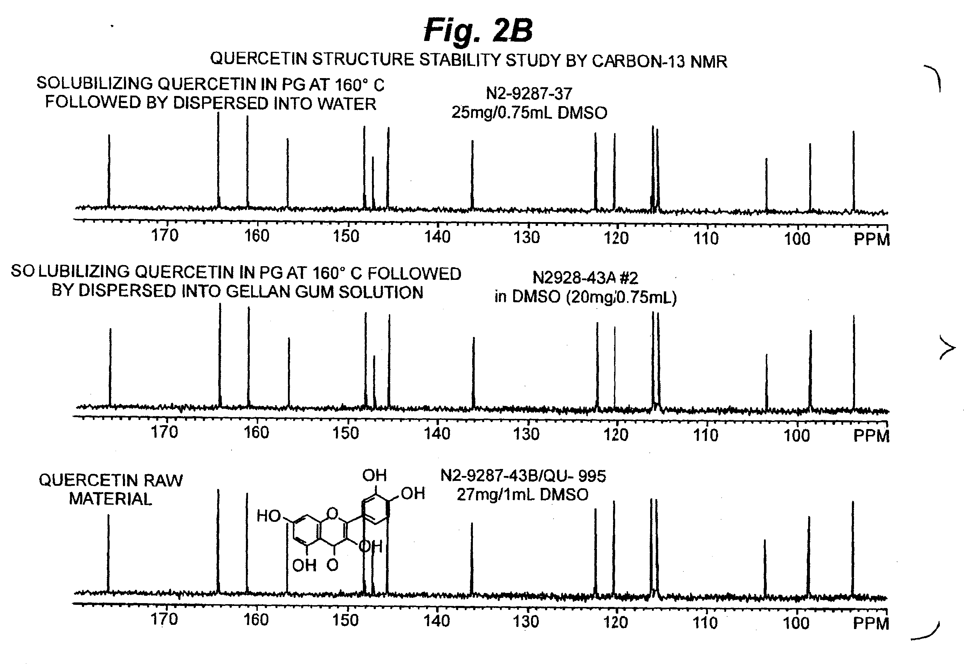

The chemical stability of quercetin during heating solubilizing process in polyols is determined by analytical characterization. Quercetin dispersion with or without gellan gum is isolated and purified from beverage. Figs. 2A and 2B show that the spectra of FT-IR and carbon-13 NMR, respectively, of quercetin dispersions through solubilization are identical with that of starting material quercetin. The results indicate that there is no structure change of quercetin during heating solubilization process.

Quercetin is solubilized by dissolving quercetin in a) hot alcohol, such as hot ethanol or polyols, or b) an alkaline solution having a pH above 7 or c) a mixture of alcohol and alkaline solution, to form solubilized quercetin solution having 1 to 15 wt % quercetin. Suitable alcohols include, but not limited to, ethanol, isopropyl alcohol, isobutyl alcohol, and benzyl alcohol, and polyols such as glycerol and propylene glycol. Combinations of alcohols are also contemplated. Preferably ethanol is used in combination with an alkaline solution of quercetin.

When completely dissolved, the quercetin solution either in hot alcohol or alkaline is added into an aqueous solution containing a stabilizer with high shear mixing or agitation. The resulting quercetin comes out of solution resulting in fine particle dispersion, suspension. The alcohol concentration is generally between 85-99 wt. %.

In addition to solubilization, quercetin can be dispersed into hot alcohol, such as hot ethanol or polyol, resulting in alcohol dispersion slurry in order to reduce quercetin particle size. Suitable alcohols include, but not limited to, ethanol, isopropyl alcohol, isobutyl alcohol, and benzyl alcohol, and polyols such as glycerol and propylene glycol. Combinations of alcohols arc also contemplated.

Stable quercetin dispersion is obtained when hot alcohol dispersion slurry is added into an aqueous solution containing stabilizer under either high shear mixing or agitation conditions. The concentration of quercetin dispersion slurry in alcohol is in the range of 1-50 wt%.

The temperature of the hot alcohol for dissolving or dispersing the quercetin is between 50-200 °C, generally 65-170 °C.

The alkaline solution may be prepared from any suitable alkaline agent such as sodium hydroxide or potassium hydroxide. The pH of the alkaline solution is above 7, generally between 10 and 12.

After the quercetin is solubilized or dispersed, the quercetin is added to a solution containing at least one dispersion stabilizer. The solution is mixed or agitated to form stable quercetin dispersion, in particular, the solubilized or dispersed quercetin is added into a stabilizer-containing aqueous solution under mixing/agitation to form a dispersion containing from 0.1 to 10 weight % quercetin. The dispersion stabilizer is present in an amount sufficient to suspend the microparticulated quercetin in the beverage.

The aqueous solution containing dispersion stabilizer is maintained at a temperature below 35 °C and at pH less than 7, typically 3 to 5, during the addition of the solubilized alkaline quercetin solution. When quercetin is solubilized or dispersed in hot alcohol, the solution or dispersion slurry is cooled between 50-130 °C prior to addition to stabilizer-containing aqueous solution.

Subsequently, water is added to dilute the concentrated dispersion to yield a stable quercetin dispersion having a concentration between 0.001 - 5.0 wt. %. The pH of the diluted dispersion is adjusted to less than 7.0, typically 2.5 to 6.

The at least one dispersion stabilizer can be a biopolymer or a modified polysaccharide such as gellan gum, pectin, carrageenan, ghatti gum, acacia gum, guar gum, xanthan gum, locust gum, agar, starch, alginate, cellulose, protein, hydrolyzed protein, modified starch, carboxyl methyl cellulose (CMC) or the combination thereof.

Preferably the biopolymers are charged polymers such as carboxyl-containing polymers and sulfate-containing polymers. It was discovered that anionic or cationic biopolymers such as pectin, gellan gum, carrageenan, gum arabic, glatti gum, CMC, whey protein isolate showed better dispersion stability than non-ionic polymers. It is believed that quercetin absorbed on a charged polymer exhibits stable aqueous dispersion due to electrostatic, steric repulsion between the particles. There is no settling/aggregation after stored at ambient conditions. The inventors discovered that the length of time that the quercetin stays dispersed in the liquid media varies depending on the type(s) of dispersion stabilizer(s) used. For example, the quercetin can stay dispersed in a beverage for 12 hours to six months or longer, depending on the dispersion stabilizer(s) used.

Controlling pH during the process is critical to make stable dispersions and to suppress undesired color development. When an alkaline solution of quercetin is added into the stabilizer-containing aqueous solution under high shear mixing, pH of the dispersion should be maintained below 6, typically below 5, or 2.5 to 4. Otherwise, if the dispersion pH is alkaline, a dark brown color is developed and does not disappear even if the pH is lowered.

The quercetin is microparticulated. "Microparticulated" or "microparticulate" as used in the instant application means a small particle ranging in size from 0.1 *u*m to 50 *u*m with an average particle size below 10 *u*m.

The microparticulated quercetin has an average particle size less than 10 microns, in particular less than 3 microns or less than 1 micron. For example, at least 90% of the particles have a particle size less than 50 microns and 80% of the particles have a particle size less than 3 microns.

The stability of quercetin aqueous dispersion is further improved by adding an organic compound with density less than 1.0 grams per cubic centimeter into an alcohol solution during solubilization of quercetin. Organic compound can be flavor ingredient such as limonene, terpene, citral, ethyl butyrate, ethyl acetate or oil such as coconut, cotton seed, olive, corn, palm, peanut, rapeseed, safflower, sesame, soja beans, sunflower, canola or combinations thereof. The concentration of organic compound in alcohol is from 0.001 to 50 wt. %.

As noted, the description focused on quercetin for ease of discussion; however, other water insoluble bioactive compounds were also investigated using the present invention dispersion method. Such bioactive compounds included curcumin, rutin, resveratrol, naringenin, hesperedin, tetramethoxyflavone (PMF), and artemisinin (anti malaria drug).

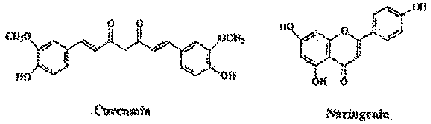

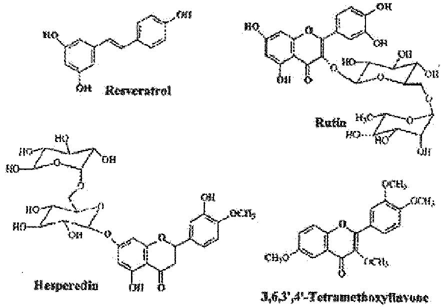

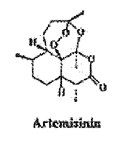

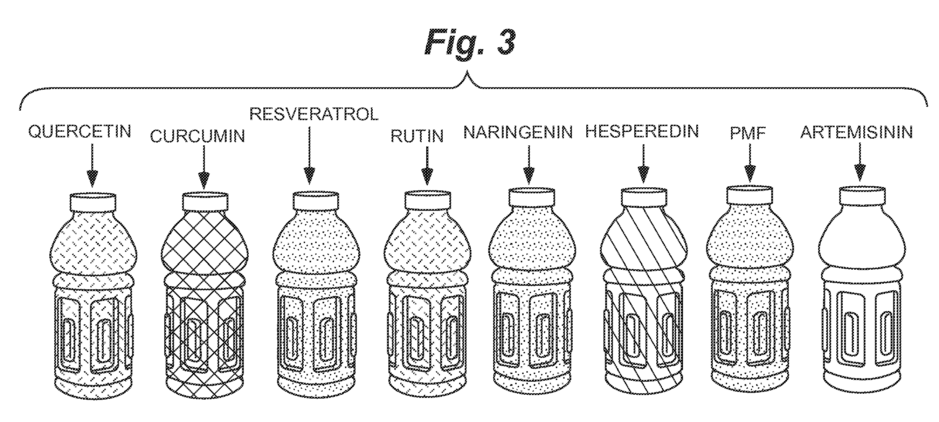

As set forth in the examples below, stable aqueous dispersions containing bioactive compounds were prepared by using the solubilization and stabilization process. The resulting dispersions were fine, homogeneous, and stable. There were no settling, phase separation, and precipitates on the side of the bottle (See Fig. 3 and Table 1). The results indicate that the presented dispersion method could be widely used to make stable water insoluble compound aqueous dispersion in different application areas.

**Table I**

| **Water-Insoluble Bioactive Compounds Aqueous Dispersion in 20 oz Beverage** | | |
|---|---|---|
| **Compound** | **Amount (mg)** | **Shelf Life (22-25 °C)** |
| Quercetin | 1000 | Stable after at least 6 months |
| Curcumin | 500 | Stable after at least 3 months |
| Rutin | 500 | Stable after at least 3 months |
| Resveratrol | 500 | Stable after at least 3 months |
| Naringenin | 500 | Stable after at least 3 months |
| Hesperedin | 500 | Stable after at least 3 months |
| Tetramethoxyflavone | 500 | Stable after at least 3 months |
| Artemisinin | 100 | Stable after at least 3 months |

The beverage may be any suitable beverage including, but not limited to, juices, carbonated soft drinks, water, dairy and isotonic beverages. One of ordinary skill in the art of the chemical and food sciences would recognize that any flavonoid may be used in accordance with the present invention.

Applicants also discovered that although the beverage appeared relatively viscous and/or thick upon adding the quercetin (or other water-insoluble bioactive compounds) to the beverage, by using the solubilized/dispersed quercetin in conjunction with the dispersion stabilizer, as disclosed herein, the beverage had a thin consistency upon consumption. This unexpected result, the thin consistency notwithstanding the viscous visual appearance, is also advantageous, as it leads to consumer likability and acceptance of the beverage.

Food-grade preservatives and acidulants may also be added to the solubilized water-insoluble bioactive compounds. Incorporating solubilized/dispersed water-insoluble bioactive compounds into the beverage results in improved suspension and dispersibility. It was further discovered that by incorporating the solubilized/dispersed water-insoluble bioactive compounds into the beverage, along with the dispersion stabilizer, that low concentration of the dispersion stabilizer may be added with a high concentration of water-insoluble bioactive compounds, and even though the water-insoluble bioactive compound is present in high concentrations and the beverage may look viscous, upon consumption it tastes thin and is easily ingested by the consumer. any other form, singularly or in combination thereof and characteristically has a sweetness potency greater than sucrose, fructose, or glucose, yet has fewer calories. Non-limiting examples of NHPS's suitable for embodiments of this invention include rebaudioside A, rebaudioside B, rebaudioside C (dulcoside B), rebaudioside D, rebaudioside E, rebaudioside F, dulcoside A, rubusoside, stevia, stevioside, mogroside IV, mogroside V, Luo Han Guo sweetener, siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobtain, baiyunoside, osladin, polypodoside A, pterocaryoside A, pterocaryoside B, mukurozioside, phlomisoside I, periandrin I, abrusoside A, and cyclocarioside I.

NHPS also includes modified NHPS's. Modified NHPS's include NHPS's which have been altered naturally. For example, a modified NHPS includes, but is not limited to, NHPS's which have been fermented, contacted with enzyme, or derivatized or substituted on the NHPS. In one embodiment, at least one modified NHPS may be used in combination with at least one NHPS. In another embodiment, at least one modified NHPS may be used without a NHPS. Thus, modified NHPS's may be substituted for a NHPS or may be used in combination with NHPS's for any of the embodiments described herein. For the sake of brevity, however, in the description of embodiments of this invention, a modified NHPS is not expressly described as an alternative to an unmodified NHPS, but it should be understood that modified NHPS's can be substituted for NHPS's in any embodiment disclosed herein.

As used herein, the phrase "synthetic sweetener" refers to any composition that is not found in nature and is a high potency sweetener. Non-limiting examples of synthetic sweeteners suitable for embodiments of this invention include sucralose, acesulfame potassium (acesulfame K or aceK) or other salts, aspartame, alitame, saccharin, neohesperidin dihydrochalcone, cyclamate, neotame, N-[3-(3-hydroxy-4-methoxyphenyl)propyl]-L-α-aspartyl]-L-phenylalanine 1-methyl ester, N-[3-(3-hydroxy-4-methoxyphenyl)-3-methylbutyl]-L-α-aspartyl]-L-phenylalanine 1-methyl ester, N-[3-(3-methoxy-4-hydroxyphenyl)propyl]-L-α-aspartyl]-L-phenylalanine 1-methyl ester, and salts thereof.

The method described herein is advantageous as it successfully suspends water-insoluble bioactive compounds such as quercetin in the beverage and thereby deters settling of the water-insoluble bioactive compounds to the bottom of the beverage's packaging.

The present application also relates to compositions comprising microparticulated flavonoid and at least one dispersion stabilize wherein 90% of the microparticulated flavonoid is below 50 microns and the at least one dispersion stabilizer is present in an amount sufficient to suspend the microparticulated flavonoid in a liquid medium, as defined in claim 15

The stability of water-insoluble bioactive compound aqueous dispersion is further improved by adding an organic compound with density less than 1.0 grams per cubic centimeter into alcohol solution during solubilization of the water-insoluble bioactive compound. Suitable organic compounds can be flavor ingredient such as limonene, terpene, citral, ethyl butyrate, ethyl acetate or oil such as coconut, cotton seed, olive, corn, palm, peanut, rapeseed, safflower, sesame, soja beans, sunflower, canola or the combination. The concentration of organic compound in alcohol is between 0.1-30 wt. %.

The following examples are specific embodiments of the present invention, but arc not intended to limit the invention.

### Example 1

A quercetin solution was prepared by dissolving 1.0 g quercetin to 20 g glycerol at temperature 150 °C. Then the solubilized quercetin solution was slowly added to an aqueous solution containing gellan gum under high mixing at pH 5. A homogeneous dispersion containing 0.5% quercetin was obtained.

The concentrated quercetin dispersion was added to the beverage and suspended in the beverage. Additional ingredients were added in the concentrations (w/w) listed below to make an isotonic beverage containing quercetin. The pH was 3.5. The pH range of the resultant isotonic beverage may be 2.5-4.5.

| **Ingredient** | **Amount (% by wt.)** |
|---|---|
| Water | 92.16% |
| Liquid Sucrose | 2.963% |
| Salt Blend | 0.176% |
| Quercetin | 0.2% |
| Gellan gum | 0.03% |
| Citric Acid | 0.180% |
| Glycerol | 4% |
| Mango Flavor | 0.100% |
| Yellow #6 Color 10% solution | 0.060% |
| Liq. Sucralose (25%) | 0.021% |
| Ace K | 0.003% |
| Vitamin C (Ascorbic Acid) | 0.105% |
| Vitamin B3 (Niacinamide) | 0.004% |
| Total | 100.000% |

### Example 2

Quercetin (1.0 g) was dissolved in 20 g propylene glycol at 150 °C. Then, quercetin solution was slowly added into gellan gum-containing aqueous solution under high shear mixing at pH 5. The quercetin dispersion was added to beverage. Additional ingredients were added in the concentrations (w/w) listed below to make an isotonic beverage containing quercetin. The pH range of the resultant isotonic beverage was 3.5.

| **Ingredient** | **Amount (% by wt.)** |
|---|---|
| Water | 92.16% |
| Liquid Sucrose | 2.963% |
| Salt Blend | 0.176% |
| Quercetin | 0.2% |
| Gellan gum | 0.03% |
| Citric Acid | 0.180% |
| Propvlene Glycol | 4% |
| Mango Flavor | 0.100% |
| Yellow #6 Color 10% solution | 0.060% |
| Liq. Sucralose (25%) | 0.021% |
| Ace K | 0.003% |
| Vitamin C (Ascorbic Acid) | 0.105% |
| Vitamin B3 (Niacinamide) | 0.004% |
| Total | 100.000% |

### Example 3

Quercetin (1.0 g) was dissolved in 10 g glycerol and 10 g propylene glycol at 150 °C. Then, quercetin polyol solution was slowly added into gellan gum-containing aqueous solution under high mixing at pH 5. The quercetin dispersion was added to beverage. Additional ingredients were added in the concentrations (w/w) listed below to make an isotonic beverage containing quercetin. The pH range of the resultant isotonic beverage was 3.5.

| **Ingredient** | **Amount (% by wt.)** |
|---|---|
| Water | 92.16% |
| Liquid Sucrose | 2.963% |
| Salt Blend | 0.176% |
| Quercetin | 0.2% |
| Gellan gum | 0.03% |
| Citric Acid | 0.180% |
| Glycerol | 2% |
| Propylene Glycol | 2% |
| Mango Flavor | 0.100% |
| Yellow #6 Color 10% solution | 0.060% |
| Liq. Sucralose (25%) | 0.021% |
| Ace K | 0.003% |
| Vitamin C (Ascorbic Acid) | 0.105% |
| Vitamin B3 (Niacinamide) | 0.004% |
| Total | 100.000% |

### Example 4

The quercetin solution was prepared by dissolving 0.5 g quercetin into 10 g sodium hydroxide (0.5 N) at room temperature. Then, quercetin alkaline solution was slowly added to an aqueous solution containing carrageenan under homogenization at pH 5. The dispersion pH was controlled below 7 during addition of quercetin alkaline solution. A homogeneous dispersion containing 0.5% quercetin is obtained. The concentrated quercetin dispersion was added to the beverage and suspended in the beverage.

Additional ingredients, including a high intensity natural sweetener were added to create a zero calorie orange-flavored isotonic beverage. The pH of the resultant beverage was 3.5.

| **Ingredient** | **Amount (% by wt**.) |
|---|---|
| Water | 99.37% |
| Citric Acid | 0.050% |
| Phosphoric Acid | 0.016% |
| Vitamin B3 | 0.005% |
| Ascorbic Acid | 0.109% |
| Carrageenan | 0.1% |
| Quercetin | 0.1% |
| Salt Blend | 0.176% |
| Reb A | 0.025% |
| Orange Flavor | 0.050% |
| Total | 100.000% |

Alternatively, or in addition to, the salt blend listed above, the beverage may contain sea salt to produce a natural zero-caloric isotonic beverage.

### Example 5

Quercetin (0.5 g) was dispersed and partially solubilized in 2.5 g ethanol at 70 °C. Then, the partially solubilized, dispersed quercetin slurry was slowly added into ghatti gum-containing aqueous solution under homogenization at pH 5. The quercetin dispersion was added to beverage. Additional ingredients were added in the concentrations (w/w) listed below to make an isotonic beverage containing quercetin. The pH was 3.5.

| **Ingredient** | **Amount (% by wt**.) |
|---|---|
| Water | 95.59% |
| Liquid Sucrose | 2.963% |
| Ethanol | 0.5 |
| Salt Blend | 0.176% |
| Ouercetin | 0.1% |
| Ghatti Gum | 0.2% |
| Citric Acid | 0.180% |
| Mango Flavor | 0.100% |
| Yellow #6 Color 10% solution | 0.060% |
| Liq. Sucralose (25%) | 0.021% |
| Acc K | 0.003% |
| Vitamin C (Ascorbic Acid) | 0.105% |
| Vitamin B3 (Niacinamide) | 0.004% |
| Total | 100.000% |

### Example 6

Quercetin (0.5 g) was dissolved in 4 g propylene glycol at 140 °C. Then, the quercetin solution was slowly added into gum arabic-containing aqueous solution under homogenization at pH 5. The quercetin dispersion was added to beverage. Additional ingredients were added in the concentrations (w/w) listed below to make an isotonic beverage containing quercetin. The pH range of the resultant isotonic beverage was 3.5.

| **Ingredient** | **Amount (% by wt.**) |
|---|---|
| Water | 95.29% |
| Liquid Sucrose | 2.963% |
| Salt Blend | 0.176% |
| Quercetin | 0.1% |
| Gum Arabic | 0.2% |
| Propylene Glycol | 0.8% |
| Citric Acid | 0.180% |
| Mango Flavor | 0.100% |
| Yellow #6 Color 10% solution | 0.060% |
| Liq. Sucralose (25%) | 0.021% |
| Ace K | 0.003% |
| Vitamin C (Ascorbic Acid) | 0.105% |
| Vitamin B3 (Niacinamide) | 0.004% |
| Total | 100.000% |

### Example 7

The quercetin solution was prepared by dissolving 0.5 g quercetin to 10 g glycerol and 0.1 g limonene at temperature 124 °C. Then the solubilized quercetin solution was slowly added to an aqueous solution containing gum arabic under homogenization at pH 5. A homogeneous dispersion containing 0.5% quercetin, was obtained. The concentrated quercetin dispersion was added to the beverage and suspended in the beverage. Additional ingredients were added in the concentrations (w/w) listed below to make an isotonic beverage containing quercetin. The pH was 3.0.

| **Ingredient** | **Amount (% by wt**.) |
|---|---|
| Water | 94.17% |
| Liquid Sucrose | 2.963% |
| Salt Blend | 0.176% |
| Quercetin | 0.1% |
| Gum Arabic | 0.1% |
| Citric Acid | 0.180% |
| Glycerol | 2% |
| limonene | 0.02% |
| Mango Flavor | 0.100% |
| Yellow #6 Color 10% solution | 0.060% |
| Liq. Sucralose (25%) | 0.021% |
| Ace K | 0.003% |
| Vitamin C (Ascorbic Acid) | 0.105% |
| Vitamin B3 (Niacinamide) | 0.004% |
| Total | 100.000% |

### Example 8

The quercetin solution was prepared by dissolving 0.5 g quercetin to 10 g glycerol and 1.0 g canola oil at temperature 120 °C. Then the solubilized quercetin solution was slowly added to an aqueous solution containing modified starch (purity gum 2000) under homogenization at pH 5. A homogeneous dispersion containing 0.5% quercetin was obtained. The concentrated quercetin dispersion was added to the beverage and suspended in the beverage. Additional ingredients were added in the concentrations (w/w) listed below to make an isotonic beverage containing quercetin. The pH was 3.5.

| **Ingredient** | **Amount (% by wt**.) |
|---|---|
| Water | 93.59% |
| Liquid Sucrose | 2.963% |
| Salt Blend | 0.176% |
| Quercetin | 0.1% |
| Purity Gum 2000 | 0.5% |
| Citric Acid | 0.180% |
| Glycerol | 2% |
| Canola Oil | 0.2% |
| Mango Flavor | 0.100% |
| Yellow #6 Color 10% solution | 0.060% |
| Liq. Sucralose (25%) | 0.021% |
| Ace K | 0.003% |
| Vitamin C (Ascorbic Acid) | 0.105% |
| Vitamin B3 (Niacinamide) | 0.004% |
| Total | 100.000% |

### Example 9

Quercetin (0.5 g) was dissolved in 10 g glycerol at 150 °C. Then, the quercetin solution was slowly added into pectin-containing aqueous solution under high shear mixing at pH 5. The quercetin dispersion was added to beverage. Additional ingredients were added in the concentrations (w/w) listed below to make an isotonic beverage containing quercetin. The pH range of the resultant isotonic beverage was 3.5.

| **Ingredient** | **Amount (% by wt**.) |
|---|---|
| Water | 94.09% |
| Liquid Sucrose | 2.963% |
| Salt Blend | 0.176% |
| Quercetin | 0.1% |
| Pectin | 0.2% |
| Citric Acid | 0.180% |
| Glycerol | 2% |
| Mango Flavor | 0.100% |
| Yellow #6 Color 10% solution | 0.060% |
| Liq. Sucralose (25%) | 0.021% |
| Ace K | 0.003% |
| Vitamin C (Ascorbic Acid) | 0.105% |
| Vitamin B3 (Niacinamide) | 0.004% |
| Total | 100.000% |

### Example 10

Curcumin (0.5 g) was dissolved in 24 g glycerol at 150 °C. Then, curcumin solution was slowly added into gellan gum-containing aqueous solution under high shear mixing at pH 5. The curcumin dispersion was added to beverage. Additional ingredients were added in the concentrations (w/w) listed below to make an isotonic beverage containing curcumin. The pH range of the resultant isotonic beverage was 3.5.

| **Ingredient** | **Amount (% by wt.**) |
|---|---|
| Water | 92.5% |
| Sucrose | 1.93% |
| Sodium Citrate | 0.13% |
| Monopotassium Phosphate | 0.04% |
| Citric Acid | 0.187% |
| Reb A | 0.015% |
| Erythritol | 0.885% |
| Ascorbic Acid | 0.103% |
| Gellan Gum | 0.03% |
| Glycerol | 4% |
| Curcumin | 0.0833% |
| Sodium Benzoate | 0.1% |
| Total | 100.000% |

### Example 11

Rutin hydrate (0.5 g) was dissolved in 24 g glycerol at 150 °C. Then, Rutin solution was slowly added into gellan gum-containing aqueous solution under high shear mixing at pH 5. The Rutin dispersion was added to beverage. Additional ingredients were added in the concentrations (w/w) listed below to make an isotonic beverage containing Rutin. The pH range of the resultant isotonic beverage was 3.5.

| **Ingredient** | **Amount (% by wt**.) |
|---|---|
| Water | 92.5% |
| Sucrose | 1.93% |
| Sodium Citrate | 0.13% |
| Monopotassium Phosphate | 0.04% |
| Citric Acid | 0.187% |
| RebA | 0.015% |
| Erythritol | 0.885% |
| Ascorbic Acid | 0.103% |
| Gellan Gum | 0.03% |
| Glycerol | 4% |
| Rutin | 0.0833% |
| Sodium Benzoate | 0.1% |
| Total | 100.000% |

### Example 12

Resveratrol (0.5 g) was dissolved in 24 g glycerol at 150 °C. Then, resveratrol solution was slowly added into gellan gum-containing aqueous solution under high shear mixing at pH 5. The resveratrol dispersion was added to beverage. Additional ingredients were added in the concentrations (w/w) listed below to make an isotonic beverage containing resveratrol. The pH range of the resultant isotonic beverage was 3.5.

| **Ingredient** | **Amount (% by wt.)** |
|---|---|
| Water | 92.5% |
| Sucrose | 1.93% |
| Sodium Citrate | 0.13% |
| Monopotassium Phosphate | 0.04% |
| Citric Acid | 0.187% |
| Reb A | 0.015% |
| Erythritol | 0.885% |
| Ascorbic Acid | 0.103% |
| Gellan Gum | 0.03% |
| Glycerol | 4% |
| Resveratrol | 0.0833% |
| Sodium Benzoate | 0.1% |
| Total | 100.000% |

### Example 13

Artemisinin (0.1 g) was dissolved in 24 g glycerol at 150 °C. Then, artemisinin solution was slowly added into gellan gum-containing aqueous solution under high shear mixing at pH 5. The artemisinin dispersion was added to beverage. Additional ingredients were added in the concentrations (w/w) listed below to make an isotonic beverage containing artemisinin. The pH range of the resultant isotonic beverage was 3.5.

| **Ingredient** | **Amount (% by wt**.) |
|---|---|
| Water | 92.57% |
| Sucrose | 1.93% |
| Sodium Citrate | 0.13% |
| Monopotassium Phosphate | 0.04% |
| Citric Acid | 0.187% |
| Rcb A | 0.015% |
| Erythritol | 0.885% |
| Ascorbic Acid | 0.103% |
| Gellan Gum | 0.03% |
| Glycerol | 4% |
| Artemisinin | 0.017% |
| Sodium Benzoate | 0.1% |
| Total | 100.000% |

### Example 14

Naringenin (0.5 g) was dissolved in 24 g glycerol at 150 °C. Then, naringenin solution was slowly added into gellan gum-containing aqueous solution under high shear mixing at pH 5. The naringenin dispersion was added to beverage. Additional ingredients were added in the concentrations (w/w) listed below to make an isotonic beverage containing naringenin. The pH range of the resultant isotonic beverage was 3.5.

| **Ingredient** | **Amount (% by wt.)** |
|---|---|
| Water | 92.5% |
| Sucrose | 1.93% |
| Sodium Citrate | 0.13% |
| Monopotassium Phosphate | 0.04% |
| Citric Acid | 0.187% |
| Reb A | 0.015% |
| Erythritol | 0.885% |
| Ascorbic Acid | 0.103% |
| Gellan gum | 0.03% |
| Glycerol | 4% |
| Naringenin | 0.0833% |
| Sodium Benzoate | 0.1% |
| Total | 100.000% |

### Example 15

Hesperedin (0.5 g) was dissolved in 24 g glycerol at 150 °C. Then, hesperedin solution was slowly added into gellan gum-containing aqueous solution under high shear mixing at pH 5. The hesperedin dispersion was added to beverage. Additional ingredients were added in the concentrations (w/w) listed below to make an isotonic beverage containing hesperedin. The pH range of the resultant isotonic beverage was 3.5.

| **Ingredient** | **Amount (% by wt.)** |
|---|---|
| Water | 92.5% |
| Sucrose | 1.93% |
| Sodium Citrate | 0.13% |
| Monopotassium Phosphate | 0.04% |
| Citric Acid | 0.187% |
| Reb A | 0.015% |
| Erythritol | 0.885% |
| Ascorbic Acid | 0.103% |
| Gellan Gum | 0.03% |
| Glycerol | 4% |
| Hesperedin | 0.0833% |
| Sodium Benzoate | 0.1% |
| Total | 100.000% |

### Example 16

3,6,3',4'-Tetramethoxyflavone (0.5 g) was dissolved in 24 g glycerol at 150 °C. Then, tetramethoxyflavone solution was slowly added into gellan gum-containing aqueous solution under high shear mixing at pH 5. The tetramethoxyflavone dispersion was added to beverage. Additional ingredients were added in the concentrations (w/w) listed below to make an isotonic beverage containing tetramethoxyflavone. The pH range of the resultant isotonic beverage was 3.5.

| **Ingredient** | **Amount (% by wt.)** |
|---|---|
| Water | 92.5% |
| Sucrose | 1.93% |
| Sodium Citrate | 0.13% |
| Monopotassium Phosphate | 0.04% |
| Citric Acid | 0.187% |
| RebA | 0.015% |
| Erythritol | 0.885% |
| Ascorbic Acid | 0.103% |
| Gellan Gum | 0.03% |
| Glycerol | 4% |
| Tetramethoxyflavone | 0.0833% |
| Sodium Benzoate | 0.1% |
| Total | 100.000% |

## Claims

1. A method for dispersing and suspending microparticulated water insoluble bioactive compound particles in a beverage, said method comprising the steps of
a) solubilizing water insoluble bioactive compound in a hot alcohol solution, an alkaline solution, or combination thereof, or dispersing the water insoluble bioactive compound in a hot alcohol solution; and
b) introducing the solubilized or dispersed bioactive compound into an aqueous solution containing at least one dispersion stabilizer; wherein the dispersion stabilizer is present in an amount sufficient to suspend the water insoluble bioactive compound in the beverage, and wherein the suspended water insoluble bioactive compound comprises microparticulated particles.

2. The method of claim 1 wherein the water insoluble bioactive compound is a polyphenol, a flavonoid such as flavonones, flavones, dihydroflavonols, flavonols, flavandiols, leucoanthocyanidins, flavonol glycosodes, flavanone glycosides, isoflavonoids, or neoflavonoids, or is extracted from plants, herbs, or botanicals.

3. The method of claims 1 or 2 wherein the water insoluble bioactive compound is selected from group consisting quercetin, eriocitrin, neoeriocitrin, narirutin, naringin, hesperidin, hesperetin, neohesperidin, neoponcirin, poncirin, rutin, isorhoifolin, rhoifolin, diosmin, neodiosmin, sinensetin, nobiletin, tangeritin, catechin, catechin gallate, epigallocatechin, epigallocatechin gallate, oolong tea polymerized polyphenol, anthocyanin, heptamethoxyflavone, daidzin, daidzein, biochaminn A, prunetin, genistin, glycitein, glycitin, genistein, 6,7,4' trihydroxy isoflavone, morin, apigenin, vitexin, balcalein, apiin, cupressuflavone, datiscetin, diosmetin, fisetin, galangin, gossypetin, geraldol, hinokiflavone, primuletin, pratol, luteolin, myricetin, orientin, robinetin, quercetagetin, and hydroxy-4-flavone, preferably
wherein the water insoluble bioactive compound is selected from the group consisting of quercetin, curcumin, rutin, resveratrol, naringenin, hesperidin , tetramethoxyflavone (PMF), and artemisinin.

4. The method of any of claims 1-3 wherein the at least one dispersion stabilizer is an anionic or cationic biopolymer or a modified polysaccharide selected from the group consisting of gellan gum, pectin, guar gum, xanthan gum, acacia gum, locust gum, agar, starch, ghatti gum, carrageenan, alginate, cellulose, protein, hydrolyzed protein, modified starch, carboxyl methyl cellulose, (CMC) whey protein isolate, and combinations thereof.

5. The method of any of claims 1-4 wherein the at least one dispersion stabilizer is present in the beverage a concentration of 0.001-5. 0 wt. % based on total weight of the beverage.

6. The method of any claims 1-5 wherein the beverage has a pH of less than 6, or 2.5 to 4.5.

7. The method of any claims 1-6 wherein the alcohol is a polyol or is selected from the group consisting of ethanol, benzyl alcohol, isopropyl alcohol, isobutyl alcohol, glycerol, and propylene glycol.

8. The method of any of claims 1-7 wherein the water insoluble bioactive compound is solubilized a hot glycerol solution, hot propylene glycol solution, or hot ethanol solution, or is solubilized in an alkaline solution at a pH of from 10 to 12.

9. The method of any of the claims 1-8 wherein the alkaline solution comprises sodium hydroxide or potassium hydroxide.

10. The method of any of claims 1-9 wherein the hot alcohol solution further comprises an organic compound selected from the group consisting of limonene, terpene, citral, ethyl butyrate, ethyl acetate, coconut oil, cotton seed oil, olive oil, corn oil, palm oil, peanut oil, rapeseed oil, safflower oil, sesame oil, soja beans oil, sunflower oil, canola oil, and combinations thereof, wherein the concentration of the organic compound in the alcohol solution is from 0.1 to 30 wt. %.

11. The method of any of claims 1-10 wherein the beverage further comprises at least one additional ingredient selected from the group consisting of carbohydrates, salts, salt blends, food-grade acids, flavors, colors, Vitamin B3, Vitamin C, sweeteners and combinations thereof, or further comprises salt in a concentration of 0.1-0.3 wt% based on the total weight of the beverage and carbohydrate in a concentration of 1-3 wt% based on the total weight of the beverage, or further comprises at least one sweetener selected from the group consisting of stevia, monatin and combinations thereof.

12. The method of any of claims 1-11 wherein the hot alcohol solution is at a temperature of 50 to 200 °C.

13. A method for suspending flavonoid particles in a beverage, said method comprising combining an aqueous solution containing at least one dispersion stabilizer and solubilized or dispersed microparticulated flavonoid, wherein the at least one dispersion stabilizer is present in an amount sufficient to suspend the microparticulated flavonoid in the beverage, and wherein the microparticulated flavonoid is solubilized or dispersed in hot glycerol, hot propylene glycol, hot ethanol, an alkaline solution, or combination thereof and wherein the flavonoid is quercetin or wherein the at least one dispersion stabilizer is an anionic or cationic biopolymer or is a gellan gum, gum arabic, pectin, carrageenan, ghatti gum, alginate, carboxyl methyl cellulose (CMC), whey protein isolate, or combinations thereof, and wherein the hot alcohol solution is at a temperature of 50 to 200 °C.

## Patentansprüche

1. Verfahren zum Dispergieren und Suspendieren mikropartikulierter wasserunlöslicher bioaktiver Verbindungspartikel in einem Getränk, wobei das Verfahren umfasst die Schritte von
a) Lösen von wasserunlöslicher bioaktiver Verbindung in einer heißen alkoholischen Lösung, einer alkalischen Lösung, oder einer Kombination davon, oder Dispergieren der wasserunlöslichen bioaktiven Verbindung in einer heißen alkoholischen Lösung; und
b) Einführen der gelösten oder dispergierten bioaktiven Verbindung in eine wässrige Lösung aufweisend wenigstens einen Dispersionsstabilisator; wobei der Dispersionsstabilisator in einer ausreichenden Menge vorliegt, um die wasserunlösliche bioaktive Verbindung in dem Getränk zu suspendieren, und wobei die suspendierte wasserunlösliche bioaktive Verbindung mikropartikulierte Partikel umfasst.

2. Verfahren gemäß Anspruch 1, wobei die wasserunlösliche bioaktive Verbindung ein Polyphenol ist, ein Flavonoid ist, wie Flavonone, Flavone, Dihydroflavonole, Flavonole, Flavandiole, Leucoanthocyanidine, Flavonol Glycosodes, Flavanon Glycoside, Isoflavonoide, oder Neoflavonoide, oder extrahiert ist aus Pflanzen, Kräutern oder Krauten.

3. Verfahren gemäß Anspruch 1 oder 2, wobei die wasserunlösliche bioaktive Verbindung ausgewählt ist aus der Gruppe bestehend aus Quercetin, Eriocitrin, Neoeriocitrin, Narirutin, Naringin, Hesperidin,
Hesperetin, Neohesperidin, Neoponcirin, Poncirin, Rutin, Isorhoifolin, Rhoifolin, Diosmin, Neodiosmin, Sinensetin, Nobiletin, Tangeritin, Catechin, Catechin Gallat, Epigallocatechin, Epigallocatechin Gallat, Oolong Tee polymerisiertem Polyphenol, Anthocyanin, Heptamethoxyflavon, Daidzin, Daidzein, Biochanin A, Prunetin, Genistin, Glycitein, Glycitin, Genistein, 6, 7, 4' Trihydroxy Isoflavon, Morin, Apigenin, Vitexin, Balcalein, Apiin, Cupressuflavon, Datiscetin, Diosmetin, Fisetin, Galangin, Gossypetin, Geraldol, Hinokiflavon, Primuletin, Pratol, Luteolin, Myricetin, Orientin, Robinetin, Quercetagetin, und Hydroxy-4-Flavon,
wobei bevorzugt die wasserunlösliche bioaktive Verbindung ausgewählt ist aus der Gruppe bestehend aus Quercetin, Curcumin, Rutin, Resveratrol, Naringenin, Hesperidin, Tetramethoxyflavone (PMF), und Artemisinin.

4. Verfahren gemäß einem der Ansprüche 1 bis 3, wobei der wenigstens eine Dispersionsstabilisator ausgewählt ist aus der Gruppe bestehend aus Gellan Gummi, Pectin, Guar Gummi, Xanthan Gummi, Acacia Gummi, Locust Gummi, Agar, Stärke, Ghatti Gummi, Carrageenan, Alginat, Cellulose, Protein, hydrolysiertem Protein, modifizierter Stärke, Carboxy
Methyl Cellulose (CMC), Weizen Protein Isolat, und Kombinationen davon.

5. Verfahren gemäß einem der Ansprüche 1 bis 4, wobei der wenigstens eine Dispersionsstabilisator in dem Getränk in einer Konzentration von 0,001 bis 5,0 Gew. % basierend auf dem Gesamtgewicht des Getränks vorliegt.

6. Verfahren gemäß einem der Ansprüche 1 bis 5, wobei das das Getränk einen pH von weniger als 6, oder 2,5 bis 4,5 hat.

7. Verfahren gemäß einem der Ansprüche 1 bis 6, wobei der Alkohol ein Polyol ist oder ausgewählt ist aus der Gruppe bestehend aus Ethanol, Benzyl Alkohol, Isopropyl Alkohol, Isobutyl Alkohol, Glycerin, und Propylen Glycol.

8. Verfahren gemäß einem der Ansprüche 1 bis 7, wobei die wasserunlösliche bioaktive Verbindung in einer heißen Glycerin Lösung, einer heißen Propylen Glykol Lösung oder einer heißen Ethanol Lösung gelöst ist oder gelöst ist in einer alkalischen Lösung bei einem pH von 10 bis 12.

9. Verfahren gemäß einem der Ansprüche 1 bis 8, wobei die alkalische Lösung umfasst Natrium Hydroxid oder Kalium Hydroxid.

10. Verfahren gemäß einem der Ansprüche 1 bis 9, wobei die heiße alkoholische Lösung ferner aufweist eine organische Verbindung ausgewählt aus der Gruppe bestehend aus Limonen, Terpen, Citral, Ethyl
Butyrat, Ethyl Acetat, Kokosnuss Öl, Baumwoll Samen Öl, Oliven Öl, Mais Keim Öl, Palm Öl, Erdnuss Öl, Raps Öl, Saflor Öl, Sesam Öl, Soja Bohnen Öl, Sonnenblumen Öl, Canola Öl, und Kombinationen davon, wobei die Konzentration der organischen Verbindung in der alkoholischen Lösung 0,1 bis 30 Gew. % ist.

11. Verfahren gemäß einem der Ansprüche 1 bis 10, wobei das Getränk ferner wenigstens einen zusätzlichen Bestandteil aufweist, ausgewählt aus der Gruppe bestehend aus Kohlenhydraten, Salzen, Salzmischungen, Nahrungsmittel reinen Säuren, Geschmacksstoffen, Farbstoffen, Vitamin B3, Vitamin C, Süßstoffen und Kombinationen davon, oder ferner umfasst Salz in einer Konzentration von 0,1 bis 0,3 Gew. % basierend auf dem Gesamtgewicht des Getränks und Kohlenhydrate in einer Konzentration von 1 bis 3 Gew. % basierend auf dem Gesamtgewicht des Getränks, oder ferner umfassend wenigstens einen Süßstoff ausgewählt aus der Gruppe bestehend aus Stevia, Monatin und Kombinationen davon.

12. Verfahren gemäß einem der Ansprüche 1 bis 11, wobei die heiße alkoholische Lösung bei einer Temperatur von 50° bis 200° ist.

13. Verfahren zum Suspendieren von Flavonoid-Partikeln in einem Getränk, wobei das Verfahren umfasst
Kombinieren einer wässrigen Lösung aufweisend
wenigstens einen Dispersionsstabilisator und gelöstes oder dispergiertes mikropartikuliertes Flavonoid, wobei der wenigstens eine Dispersionsstabilisator in einer ausreichenden Menge vorliegt, um das mikropartikulierte Flavonoid in dem Getränk zu suspendieren, und wobei das mikropartikulierte Flavonoid gelöst oder dispergiert ist in heißer Glycerin-Lösung, heißer Propylen Glykol-Lösung, heißer Ethanol-Lösung, einer alkalischen Lösung oder Kombinationen davon und wobei das Flavonoid Quercetin ist, oder wobei der wenigstens eine Dispersionsstabilisator ein anionisches oder kationische Biopolymer oder ein Gellan Gummi, Gummi Arabicum, Pectin, Carrageenan, Ghatti Gummi, Alginat, Carboxy Methyl Cellulose (CMC), Weizen Protein Isolat, oder Kombinationen davon ist, und wobei die heiße alkoholische Lösung bei einer Temperatur von 50 bis 200 °C ist.

## Revendications

1. Procédé pour disperser et mettre en suspension dans une boisson des particules de composé bioactif insoluble dans l'eau sous forme de microparticules, ledit procédé comprenant les étapes consistant à
a) solubiliser le composé bioactif insoluble dans l'eau dans une solution alcoolique chaude, une solution alcaline, ou une combinaison de celles-ci, ou disperser le composé bioactif insoluble dans l'eau dans une solution alcoolique chaude ; et
b) introduire le composé bioactif solubilisé ou dispersé dans une solution aqueuse contenant au moins un stabilisant de dispersion ;
dans lequel le stabilisant de dispersion est présent en une quantité suffisante pour mettre en suspension dans la boisson le composé bioactif insoluble dans l'eau, et dans lequel le composé bioactif insoluble dans l'eau en suspension comprend des particules sous forme de microparticules.

2. Procédé selon la revendication 1, dans lequel le composé bioactif insoluble dans l'eau est un polyphénol, un flavonoïde tel que des flavonones, des flavones, des dihydroflavonols, des flavonols, des flavanediols, des leucoanthocyanidines, des flavonol-glycosides, des flavanone-glycosides, des isoflavonoïdes, ou des néoflavonoïdes, ou est extrait à partir de plantes, d'herbes, ou de produits d'origine végétale.

3. Procédé selon la revendication 1 ou 2, dans lequel le composé bioactif insoluble dans l'eau est choisi dans le groupe constitué par la quercétine, l'ériocitrine, la néoériocitrine, la narirutine, la naringine, l'hespéridine, l'hespérétine, la néohespéridine, la néoponcirine, la poncirine, la rutine, l'isorhoifoline, la rhoifoline, le diosmine, la néodiosmine, la sinensétine, la nobilétine, la tangéritine, la catéchine, le gallate de catéchine, l'épigallocatéchine, le gallate d'épigallocatéchine, les polyphénols polymérisés de thé oolong, l'anthocyanine, l'heptaméthoxyflavone, la daidzine, la daidzéine, la biochaminne A, la prunétine, la génistine, la glycitéine, la glycitine, la génistéine, le 6,7,4'-trihydroxy-isoflavone, la morine, l'apigénine, la vitexine, la balcaléine, l'apiine, la cupressuflavone, la datiscétine, la diosmétine, la fisétine, la galangine, la gossypétine, le géraldol, l'hinokiflavone, la primulétine, le pratol, la lutéoline, la myricétine, l'orientine, la robinétine, la quercétagétine, et l'hydroxy-4-flavone, de préférence dans lequel le composé bioactif insoluble dans l'eau est choisi dans le groupe constitué par la quercétine, la curcumine, la rutine, le resvératrol, la naringénine, l'hespéridine, la tétraméthoxyflavone (PMF) et l'artémisinine.

4. Procédé selon l'une quelconque des revendications 1 à 3, dans lequel l'au moins un stabilisant de dispersion est un biopolymère anionique ou cationique ou un polysaccharide modifié choisi dans le groupe constitué par la gomme gellane, la pectine, la gomme de guar, la gomme xanthane, la gomme arabique, la gomme de caroube, l'agar, l'amidon, la gomme ghatti, la carraghénane, un alginate, la cellulose, une protéine, une protéine hydrolysée, un amidon modifié, la carboxyméthylcellulose (CMC), un isolat de protéine de petit-lait, et des combinaisons de ceux-ci.

5. Procédé selon l'une quelconque des revendications 1 à 4, dans lequel l'au moins un stabilisant en dispersion est présent dans la boisson à une concentration de 0,001 à 5,0 % en poids par rapport au poids total de la boisson.

6. Procédé selon l'une quelconque des revendications 1 à 5, dans lequel la boisson a un pH inférieur à 6, ou de 2,5 à 4,5.

7. Procédé selon l'une quelconque des revendications 1 à 6, dans lequel l'alcool est un polyol ou est choisi dans le groupe constitué par l'éthanol, l'alcool benzylique, l'alcool isopropylique, l'alcool isobutylique, le glycérol, et le propylèneglycol.

8. Procédé selon l'une quelconque des revendications 1 à 7, dans lequel le composé bioactif insoluble dans l'eau est solubilisé dans une solution chaude de glycérol, une solution chaude de propylèneglycol, ou une solution chaude d'éthanol, ou bien est solubilisé dans une solution alcaline à un pH de 10 à 12.

9. Procédé selon l'une quelconque des revendications 1 à 8, dans lequel la solution alcaline comprend de l'hydroxyde de sodium ou de l'hydroxyde de potassium.

10. Procédé selon l'une quelconque des revendications 1 à 9, dans lequel la solution alcoolique chaude comprend en outre un composé organique choisi dans le groupe constitué par le limonène, le terpène, le citral, le butyrate d'éthyle, l'acétate d'éthyle, l'huile de coco, l'huile de coton, l'huile d'olive, l'huile de maïs, l'huile de palme, l'huile d'arachide, l'huile de colza, l'huile de carthame, l'huile de sésame, l'huile de soja, l'huile de tournesol, l'huile de canola, et des combinaisons de ceux-ci, et dans lequel la concentration du composé organique dans la solution alcoolique est de 0,1 à 30 % en poids.

11. Procédé selon l'une quelconque des revendications 1 à 10, dans lequel la boisson comprend en outre au moins un ingrédient additionnel choisi dans le groupe constitué par les hydrates de carbone, les sels, les mélanges de sels, les acides de qualité alimentaire, les arômes, les colorants, la vitamine B3, la vitamine C, les édulcorants et des combinaisons de ceux-ci, ou comprend en outre un sel à une concentration de 0,1 à 0,3 % en poids par rapport au poids total de la boisson et un hydrate de carbone à une concentration de 1 à 3 % en poids par rapport au poids total de la boisson, ou comprend en outre au moins un édulcorant choisi dans le groupe constitué par le stevia, la monatine et des combinaisons de ceux-ci.

12. Procédé selon l'une quelconque des revendications 1 à 11, dans lequel la solution alcoolique chaude est à une température de 50 à 200°C.

13. Procédé pour mettre des particules de flavonoïde en suspension dans une boisson, ledit procédé comprenant la combinaison d'une solution aqueuse contenant au moins un stabilisant de dispersion et un flavonoïde sous forme de microparticules solubilisées ou dispersées, dans lequel l'au moins un stabilisant de dispersion est présent en une quantité suffisante pour mettre en suspension dans la boisson le flavonoïde sous forme de microparticules, et dans lequel le flavonoïde sous forme de microparticules est solubilisé ou dispersé dans du glycérol chaud, du propylèneglycol chaud, de l'éthanol chaud, une solution alcaline, ou une combinaison de ceux-ci, et dans lequel le flavonoïde est la quercétine, ou dans lequel l'au moins un stabilisant de dispersion est un biopolymère anionique ou cationique ou est la gomme gellane, la gomme arabique, la pectine, la carraghénane, la gomme ghatti, un alginate, la carboxyméthylcellulose (CMC), un isolat de protéine de petit-lait, ou des combinaisons de ceux-ci, et dans lequel la solution alcoolique chaude est à une température de 50 à 200°C.
